# EUROPEAN PATENT SPECIFICATION

(11) **EP 2 639 789 B1**
(45) Date of publication and mention of the grant of the patent: **08.06.2016**
(21) Application number: 13159231.3
(22) Date of filing: 14.03.2013
(51) Int. Cl.: G10K 11/00, B06B 1/02

(54) **Multi-array ultrasonic probe apparatus and method for manufacturing multi-array probe apparatus**
Mehrfach-Array-Ultraschallsondenvorrichtung und Verfahren zum Herstellen der Mehrfach-Array-Sondenvorrichtung
Appareil de sonde ultrasonore multi-réseau et procédé de fabrication de l'appareil de sonde ultrasonore multi-réseau

(30) Priority: 14.03.2012 KR 20120026098
(43) Date of publication of application: 18.09.2013
(73) Proprietor: Samsung Electronics Co., Ltd, Gyeonggi-do 443-742 (KR)
(72) Inventor: Kim, Young Il, Yongin-si Gyeonggi-do 446-712 (KR); Kim, Dong Wook, Yongin-si Gyeonggi-do 446-712 (KR); Kim, Bae Hyung, Yongin-si Gyeonggi-do 446-712 (KR); Song, Jong Keun, Yongin-si Gyeonggi-do 446-712 (KR); Lee, Seung Heun, Yongin-si Gyeonggi-do 446-712 (KR); Cho, Kyung Il, Yongin-si Gyeonggi-do 446-712 (KR)
(74) Representative: Grünecker Patent- und Rechtsanwälte PartG mbB

(56) References cited:
- EP-A2- 2 610 860
- GB-A- 2 430 259
- US-A- 5 655 276
- US-A- 6 104 126
- US-A1- 2010 241 004
- US-A1- 2012 118 475

## Description

### BACKGROUND

### 1. Field

Apparatuses and methods consistent with exemplary embodiments relate to a multi-array ultrasonic probe apparatus that may provide a stable ultrasonic beam by aligning tiles in identical directions and at identical levels.

### 2. Description of Related Art

A diagnostic ultrasound system is an apparatus that may radiate, from a body surface of a target object, an ultrasonic beam toward a desired part inside a body, and may obtain a cross section of soft tissues or an image of a blood flow, using a reflected ultrasonic beam.

The diagnostic ultrasound system may include an ultrasonic probe apparatus that may obtain ultrasonic data by transmitting an ultrasonic beam to the target object and receiving an ultrasonic beam reflected from the target object.

Here, the ultrasonic probe apparatus may obtain ultrasonic data about the target object by transmitting and receiving an ultrasonic beam while moving along with the target object in contact with the ultrasonic probe apparatus. Document US 5,655,276 is directed to a method of manufacturing two-dimensional array ultrasonic transducers. An array ultrasonic transducer includes a substrate having a surface on which a plurality of pads or electrodes are formed. Document US 2012/0118475 A1 describes methods of fabricating ultrasonic transducer assemblies. The method includes applying one or more layers of patternable material to at least a portion of a surface of a wafer comprising a number of die. Document US 2010/0241004 A1 is directed at a probe for an ultrasonic diagnostic apparatus and a method of manufacturing the same. In EP 2 610 860 A2, which is a prior art document cited under Article 54(3) EPC, an ultrasound probe and a manufacturing method thereof are described. GB 2 430 259 A is concerned with an acoustic transducer with an integrated electronics module.

### SUMMARY

The invention provides a multi-array ultrasonic probe apparatus according to claim 1 and a method of manufacturing a multi-array ultrasonic probe apparatus according to claim 9. According to an aspect of an exemplary embodiment, there is provided a multi-array ultrasonic probe apparatus, including n tiles formed to transmit and receive an ultrasonic beam, with respect to a target object, and a substrate including n guide portions on which the n tiles are mounted, respectively, to be aligned in a form of a multi-array. Here, n denotes a natural number. Accordingly, the n tiles may be aligned in uniform directions, by disposing the tiles to be mounted on the guide portions collinearly in an array, for example, in an x-direction and/or a y-direction.

The substrate may further include n adhesive portions below the n guide portions, the n adhesive portions on which an adhesive material that bonds the n tiles to the substrate may be disposed. This secures the tiles on the substrate.

The substrate may further include n outlets formed to take out the excess adhesive, to an outside, the adhesive material disposed on the n adhesive portions that the n tiles may be in contact with when the n tiles are mounted on the n guide portions. This allows for the removal of excessive adhesive during mounting, thereby preventing damage of the tiles, thereby preventing the adhesive material from leaking to the tiles to prevent a malfunction of the tiles.

Widths of the n guide portions may be wider than widths of the n adhesive portions. Hence, the tiles may be in contact with the respective adhesive portions that may be disposed in a lower portion of the guide portion. Further, the tiles may be secured only in a central region thereof, for example.

The n guide portions each may have identical heights, and the n adhesive portions each may have identical heights. Accordingly, the n guide portions and the n adhesive portions may enable the leveling of the n tiles such that a time for transmitting and receiving the ultrasonic beam at the n tiles may be controlled, for example, identically.

The n guide portions may be disposed in a form of a matrix on the substrate such that a predetermined gap separates the n guide portions, and the n adhesive portions may be disposed in a form of a matrix on the substrate such that a predetermined gap separates the n adhesive portions. Accordingly, a direction for transmitting and receiving an ultrasonic beam at the tiles may be controlled, whereby an accuracy of the ultrasonic beam may be increased.

Widths of the n guide portions may be wider than widths of the n tiles by a predetermined size. Thus, the tiles may be readily inserted in the guide portion.

Each of the n tiles may include an Application Specific Integrated Circuit (ASIC), and a Capacitive Micromachined Ultrasonic Transducer (CMUT) attached to an upper portion of the ASIC. These are particularly preferred tiles for transmitting and receiving an ultrasonic beam.

The substrate may be formed of one of silicon, glass, and a polymer-based material. These are particularly suitable materials.

According to another general aspect of an exemplary embodiment, there is provided a method of manufacturing a multi-array ultrasonic probe apparatus, the method including providing a substrate including n guide portions, and aligning n tiles formed to transmit and receive an ultrasonic beam in a form of a multi-array by mounting the n tiles on the n guide portions, respectively. Here, n denotes a natural number.

The advantages of the method according to the invention and the following exemplary embodiments correspond to the respective advantages as already described in connection with the apparatus above.

According to one or more of exemplary embodiments, a multi-array ultrasonic probe apparatus may obtain more accurate ultrasonic data by mounting tiles to be aligned in identical directions and at identical levels on a substrate, thereby controlling a direction and a time for transmitting and receiving an ultrasonic beam to be transmitted and received at the tiles.

According to one or more of exemplary embodiments, a multi-array ultrasonic probe apparatus may readily align tiles in a form of a multi-array, using a substrate including guide portions, in which tiles are to be mounted, disposed in a form of a matrix such that a predetermined gap separates the guide portions.

According to one or more of exemplary embodiments, a multi-array ultrasonic probe apparatus may reduce a margin of error of guide portions and adhesive portions to be within a few micrometers (µm), using a substrate on which the guide portions and the adhesive portions are formed by semiconductor process technology, thereby uniformly aligning tiles that are to be mounted on the guide portions and to be in contact with the adhesive portions.

According to one or more of exemplary embodiments, a multi-array ultrasonic probe apparatus may be manufactured at a relatively low cost or in a relatively short period of time, using a substrate formed of a polymer-based material by imprinting technology based on a preformed substrate, for example, a silicon substrate.

According to one or more of exemplary embodiments, a multi-array ultrasonic probe apparatus may include an outlet on one side of each adhesive portion on a substrate, and may provide a path for discharging an adhesive material to an outside when the adhesive material, disposed in the adhesive portion that a tile may be in contact with, is pressed by the tile to be mounted on a guide portion, thereby preventing damage to the tiles.

### BRIEF DESCRIPTION OF THE DRAWINGS

The above and/or other aspects will become more apparent by describing certain exemplary embodiments, with reference to the accompanying drawings, in which:
FIG. 1A is a perspective view illustrating an example of a multi-array ultrasonic probe apparatus according to an exemplary embodiment.
FIG. 1B is a cross-sectional view cut along a line A-A' of FIG. 1A.
FIG. 2 illustrates an example of a multi-array ultrasonic probe apparatus according to an exemplary embodiment.
FIG. 3 is a flowchart illustrating an example of a method of manufacturing a multi-array ultrasonic probe apparatus according to an exemplary embodiment.
FIG. 4 is a top view illustrating an example of a substrate in a multi-array ultrasonic probe apparatus according to an exemplary embodiment.
FIG. 5 is a perspective view illustrating an example of inserting tiles in a multi-array ultrasonic probe apparatus according to an exemplary embodiment.
FIG. 6 is a cross-sectional view to describe an example of a method of manufacturing a substrate of a multi-array ultrasonic probe apparatus according to an exemplary embodiment.
FIG. 7 is a cross-sectional view to describe another example of a method of manufacturing a substrate of a multi-array ultrasonic probe apparatus according to an exemplary embodiment.
Throughout the drawings and the detailed description, unless otherwise described, the same drawing reference numerals will be understood to refer to the same elements, features, and structures. The relative size and depiction of these elements may be exaggerated for clarity, illustration, and convenience.

### DETAILED DESCRIPTION

Hereinafter, certain exemplary embodiments are described in detail below with reference to the accompanying drawings.

The following detailed description is provided to assist the reader in gaining a comprehensive understanding of the methods, apparatuses, and/or systems described herein. Accordingly, various changes, modifications, and equivalents of the methods, apparatuses, and/or systems described herein will be suggested to those of ordinary skill in the art. The progression of processing steps and/or operations described is an example; however, the sequence of and/or operations is not limited to that set forth herein and may be changed as is known in the art, with the exception of steps and/or operations necessarily occurring in a certain order. Also, description of well-known functions and constructions may be omitted for increased clarity and conciseness.

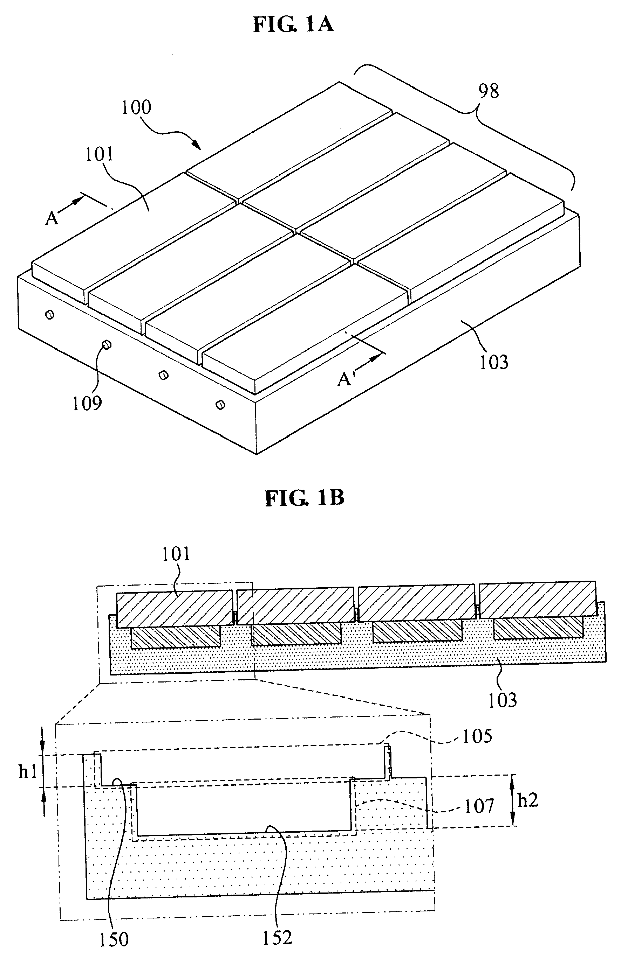

FIGS. 1A and 1B illustrate an example of a structure of a multi-array ultrasonic probe apparatus 100. In particular, FIG. 1A is a perspective view of the multi-array ultrasonic probe apparatus 100, and FIG. 1B is a cross-sectional view cut along a line A-A' of FIG. 1A.

Referring to FIGS. 1A and 1B, the multi-array ultrasonic probe apparatus 100 includes a tile 101, and a substrate 103.

For instance, n tiles 98 may be provided. Here, n denotes a natural number. Although FIG. 1A illustrates only eight tiles, any appropriate number of tiles, fewer or greater than eight, may be provided.

The tile 101 may obtain ultrasonic data by transmitting and receiving an ultrasonic beam with respect to a target object. The tile 101 may include an Application Specific Integrated Circuit (ASIC), and a Capacitive Micromachined Ultrasonic Transducer (CMUT) attached on an upper portion of the ASIC.

The substrate 103 includes n guide portions 105 and n adhesive portions 107 which correspond to n tiles 98 and are provided in a form of a matrix. The n tiles 98 may be mounted on the n guide portions 105 and may be aligned in a form of a multi-array, which may include a multiple number of one-dimensional arrays. That is, the n guide portions 105 may be attached in rows and columns on the substrate 103 such that a predetermined gap separates the n guide portions 105. Also, the n adhesive portions 107 may be arranged in rows and columns on the substrate 103 such that a predetermined gap separates the n adhesive portions 107. Accordingly, the n tiles 98 may be aligned in uniform directions, by disposing the n tiles 98 to be mounted on the n guide portions 105 collinearly, for example, in an x-axial direction or a y-axial direction.

In particular, the n guide portions 105 may be provided on an upper surface 150 of the substrate 103 so that the n tiles 98 may be mounted on the n guide portions 105, respectively. Further, the n adhesive portions 107 may be provided on a lower surface 152 of the substrate 103 at positions corresponding to the positions of the n guide portions 105, respectively. An adhesive material, for example epoxy, may be disposed in the adhesive portions 107 to bond the n tiles 98 to the substrate 103.

Also, the substrate 103 may include an outlet 109 on one side of one or more of the adhesive portions 107. Accordingly, when the adhesive material, disposed in the respective adhesive portion 107 is pressed by the tile 101 to be mounted in the respective guide portion 105, the adhesive material may be prevented from leaking in a direction of the tile 101 by discharging the adhesive material to an outside through the respective outlet 109, thereby preventing damage to the tile 101.

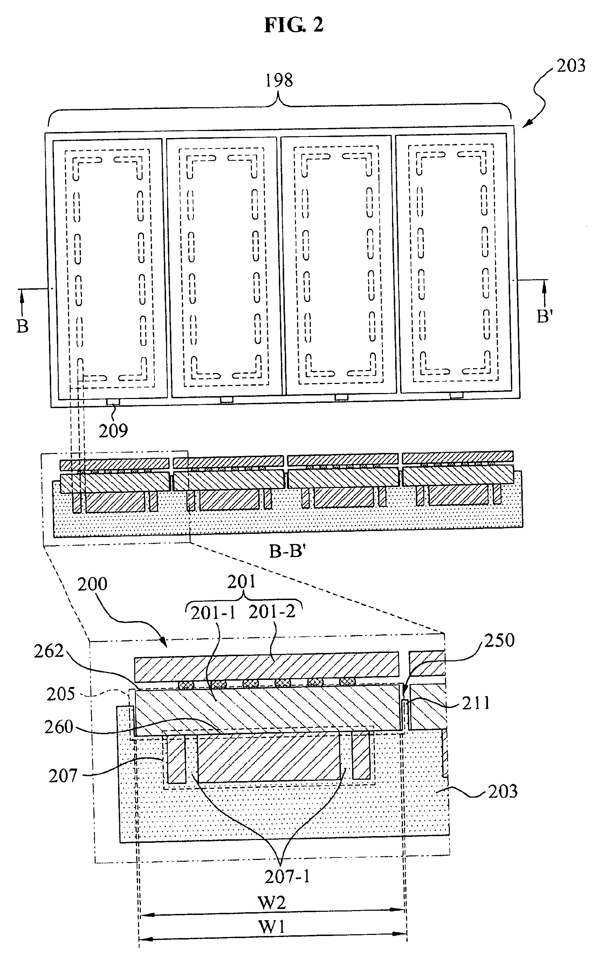

FIG. 2 illustrates an example of a multi-array ultrasonic probe apparatus 200. In particular, an uppermost diagram is a top view of the multi-array ultrasonic probe apparatus 200, and a lowermost diagram is a cross-sectional view cut along a line B-B'.

Referring to FIG 2, the multi-array ultrasonic probe apparatus 200 includes a tile 201, and a substrate 203.

For instance, n tiles 198 may be provided. Here, n denotes a natural number. The tile 201 may transmit and receive an ultrasonic beam with respect to a target object. The tile 201 includes an ASIC 201-1, with a lower portion 260 disposed on a corresponding adhesive portion 207, and a CMUT 201-2 disposed on an upper portion 262 of the ASIC 201-1. For instance, the CMUT 201-2 may be attached to the ASIC 201-1 by flip chip bonding technology.

As an example, the substrate 203 may be formed of silicon, glass, or a polymer-based material. The substrate 203 includes guide portions 205 and adhesive portions 207, which correspond to n tiles 198. An outlet or outlets 209 may be disposed on one or both sides of one or more of the adhesive portions 205.

The substrate 203 may be formed by semiconductor process technology or imprinting technology. As an example, when the guide portion 205 and the adhesive portion 207 are formed on the substrate 203 by the semiconductor process technology, a margin of error of the guide portion 205 and the adhesive portion 207 may be reduced to be within a few micrometers (µm). Accordingly, the tiles 198 to be mounted in the respective guide portions 205 and to be in contact with adhesive portions 207 may be aligned uniformly. As another example, the substrate 203 is formed of the polymer-based material by the imprinting technology using a preformed substrate, for example a silicon substrate. Accordingly, the substrate 203 may be formed at a relatively low cost or in a relatively short period of time.

The n guide portions 205 corresponding to a number of the tiles 198 may be provided. The n tiles 198 may be mounted on the n guide portions 205, respectively, to be aligned in a form of a multi-array. The respective guide portion 205 may be provided in a shape identical to an outline of the tile 201 so that the tile 201 may be readily inserted and mounted on the guide portion 205. For example, the guide portion 205 may be formed to have inner corners provided at right angles, for example, in an L-shape and a mirrored L-shape. Accordingly, the tile 201 may be mounted in the guide portion 205 such that a portion of a lower end of the tile 201, and a portion of a side of the tile 201 may be in contact with the guide portion 205, simultaneously.

Also, by forming the guide portion 205 to have a width w1 wider than a width w2 of the tile 201. by a predetermined size, for example, 10 to 20 micrometers (µm), the tile 201 may be readily inserted in the guide portion 205. The tile 201 may be mounted in a central portion of the guide portion 205 such that a predetermined gap 250 may be maintained on both sides of the tile 201. For example, when the width w1 of the guide portion 205 is wider than the width w2 of the tile 201 by 10 µm, a 5 µm gap may be maintained on a left side between the guide portion 205 and one side of the tile 201, and a 5 µm gap may be maintained on a right side between the guide portion 205 and another side of the tile 201, as seen in a lower part of FIG. 2.

The adhesive portion 207 may be formed on or proximate to a lower portion of the respective guide portion 205, and an adhesive material, for example epoxy, may be disposed in the adhesive portion 207 to bond the tile 201 to the substrate 203.

The n guide portions 205 may be disposed in a form of a matrix on the substrate 203 such that a predetermined gap, for example, a 20 µm gap, separates the n guide portions from one another. The n adhesive portions 207 may be disposed in a form of a matrix on the substrate 203 such that a predetermined gap, for example, a 20 µm gap, separates the n adhesive portions 207 from one another. The n tiles 198 to be mounted in the guide portions 205, respectively, may be disposed collinearly, for example, in an x-axial direction or a y-axial direction, whereby the n tiles 198 may be aligned in uniform directions. That is, the directions of the n tiles 198 may be aligned by the n guide portions 205 and the n adhesive portions 207. Accordingly, a direction for transmitting and receiving an ultrasonic beam at the tiles 198 may be controlled, whereby an accuracy of the ultrasonic beam may be increased.

The guide portion 205 may be formed to have a width wider than a width of the corresponding adhesive portion 207 so that the tile 201 may be in contact with the adhesive portion 207 disposed in a lower portion of the guide portion 205, and a portion in which the tile 201 may be mounted stably may be secured.

The n guide portions 205 may each have identical heights h1, and the n adhesive portions 207 may each have identical heights h2. For example, the n guide portions 205 may each be formed to have identical heights in a range of tens of µm to hundreds of µm. Also, the n adhesive portions 207 each may be formed to have identical heights in a range of tens of µm to hundreds of µm.

When each of the n guide portions 205 is formed to have identical heights, and each of the n adhesive portions 207 is formed to have identical heights, the tiles 198 may be mounted on the substrate 203 at identical heights, whereby even leveling of the tiles 198 may be supported and provided. Accordingly, the n guide portions 205 and the n adhesive portions 207 may enable the leveling of the tiles 198 such that a time for transmitting and receiving the ultrasonic beam at the n tiles 198 may be controlled, for example, identically.

That is, the n guide portions 205 and the n adhesive portions 207 may enable the n tiles 198 to have identical heights. Accordingly, when each of the tiles 198 transmits an ultrasonic beam to a target object at identical times, and a feedback ultrasonic beam arrives from the target object at identical times, the feedback ultrasonic beam may be received or detected at identical times.

However, the heights of the guide portions 205 or the heights of the adhesive portions 207 may be identical or different.

At least one of the adhesive portions 207 may include a first projection 207-1, for example, a column-shaped projection, to reduce a movement of the adhesive portion 207 resulting from oscillation of the corresponding tile 201 occurring during transmission and reception of an ultrasonic beam, thereby bonding the tile 201 to the substrate 203 more stably. As shown in FIG. 2, two first projections 207-1 are formed, but this is not limiting and any appropriate number of projections may be formed.

Outlets 209 may be provided on one side or both sides of the n adhesive portions 207. When the adhesive material, disposed in the respective adhesive portion 207 is pressed by the tile 201 to be mounted in the guide portion 205, the adhesive material may be discharged to an outside. That is, when the tile 201 is inserted in the respective guide portion 205, the outlet 209 may provide a path for discharging the adhesive material disposed in the respective adhesive portion 207 to the outside of the structure, thereby preventing the adhesive material from leaking to the tile 201 to prevent a malfunction of the tile 201.

The substrate 203 further includes a second projection 211 which is disposed between the adjacent guide portions 205 and has sides proximate to adjacent guide portions 205, respectively, to separate the adjacent guide portions 205. The second projection 211 may be formed to have, for example, a height and a width in a range of tens of µm.

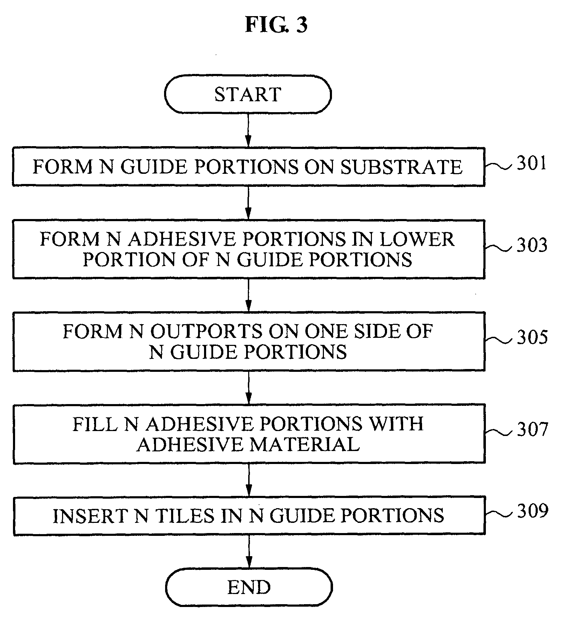

FIG. 3 illustrates an example of a method of manufacturing a multi-array ultrasonic probe apparatus.

Referring to FIG. 3, in operation 301, n guide portions are formed, on a substrate, to mount n tiles, respectively.

The substrate may include a polymer substrate, a Silicon-on-Insulator (SOI) substrate, a substrate formed of at least one semiconductor material including silicon (Si), germanium (Ge), silicon germanium (SiGe), gallium phosphide (GaP), gallium arsenide (GaAs,) silicon carbide (SiC), silicon germanium carbide (SiGeC), indium arsenide (InAs), and indium phosphide (InP), and the like. However, the semiconductor material is not limited thereto.

The n guide portions may be disposed in a form of a matrix on the substrate such that a predetermined gap, for example, a 20 µm gap, separates the n guide portions. That is, the n guide portions may enable the n tiles to be disposed collinearly, for example, in an x-axial direction or a y-axial direction, so that the n tiles may be aligned in uniform directions. Accordingly, a direction for transmitting and receiving an ultrasonic beam at the tiles may be controlled, whereby an accuracy of the ultrasonic beam may be increased.

When the n guide portions are formed to have identical heights in the range of tens of µm to hundreds of µm, leveling of the n tiles to be mounted in the n guide portions may be supported.

A guide portion may be formed to have a width wider than a width of a tile by a predetermined size, for example, 10 µm to 20 µm, whereby the tile may be readily inserted.

Also, when the n guide portions are formed, a second projection, for example, a column-shaped projection, may be formed on the substrate to separate adjacent guide portions. That is, the second projection may correspond to a portion remaining between the adjacent guide portions, without being etched, during a process of forming the n guide portions, for example, by an etching process. For example, the second projection may be formed to have a height and a width of tens of µm.

In operation 303, n adhesive portions are formed in a lower portion of the guide portions. An adhesive material, for example epoxy, may be disposed in the n adhesive portions to bond the n tiles to the substrate.

The n adhesive portions may be disposed in a form of a matrix in the lower portion of the guide portions, at positions corresponding to positions of the n guide portions. For example, the n adhesive portions may be formed to have identical heights in the range of tens of µm to hundreds of µm.

An adhesive portion may be formed to have a width narrower than a width of a guide portion by a predetermined size such that a tile to be mounted in the guide portion may be in contact with the adhesive portion while a gap for mounting the tile stably may be secured in the guide portion.

Also, the adhesive portion may include a first projection, for example, a column-shaped projection, thereby reducing a movement of the adhesive portion resulting from oscillation generated by the tile to be mounted in the guide portion and to be in contact with the adhesive portion during transmission and reception of an ultrasonic beam. Accordingly, the tile may be bonded to the substrate more stably.

In operation 305, n outlets are formed, on one side of the n adhesive portions, to discharge the adhesive material to be disposed in the adhesive portion to an outside.

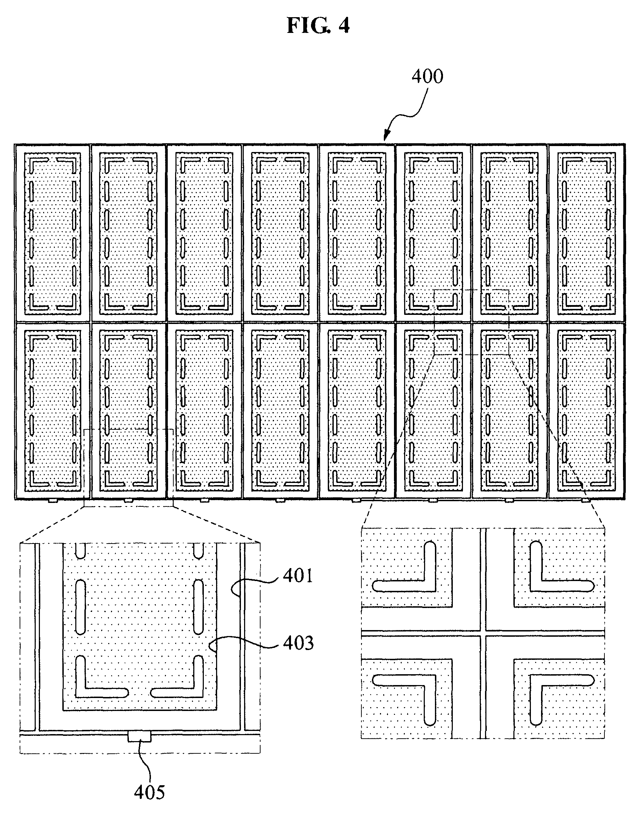

For instance, the substrate may be formed, as shown in FIG. 4. Although the outlets are formed on one side of the adhesive portions, the positions of the outlets are not limited thereto. For example, the outlets may be formed on both sides facing each other, whereby the adhesive material may be discharged to the outside on both sides. Referring to FIG. 4, the substrate 400 includes n guide portions 401, n adhesive portions 403, and n outlets 405.

In operation 307, the n adhesive portions are filled with the adhesive material, for example, epoxy.

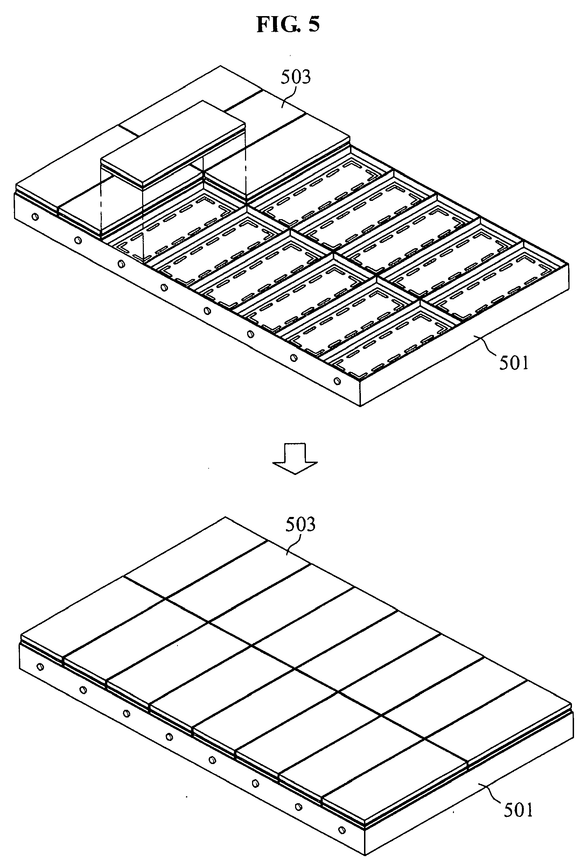

In operation 309, the n tiles are inserted in the n guide portions, respectively. For instance, the n tiles may be mounted on the substrate, as shown in FIG. 5. An individual tile may correspond to a chip on which an ASIC and a CMUT may be laminated sequentially.

As shown in FIG. 5, a multi-array ultrasonic probe apparatus may be formed by mounting n tiles 503 on a substrate 501, sequentially.

For instance, the outlets formed on one side of the adhesive portions may discharge the adhesive material to an outside when the adhesive material is pressed by the tiles to be mounted in the guide portions and simultaneously, to be in contact with the adhesive material disposed in the adhesive portions disposed in a lower portion of the guide portions. That is, an outlet may provide a path for discharging the adhesive material to the outside when the tiles are mounted in the guide portion, thereby preventing the adhesive material from leaking to the tile, and preventing damage to the tile.

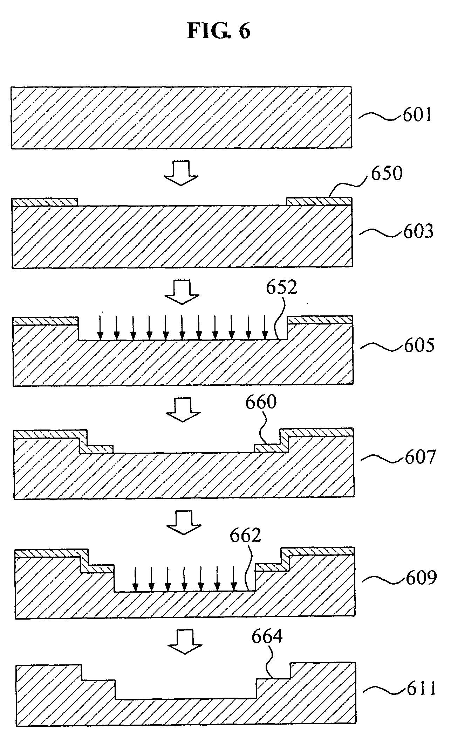

FIG. 6 illustrates an example of a method of manufacturing a substrate of a multi-array ultrasonic probe apparatus.

Referring to FIG. 6, in operation 601, a substrate is provided. As an example, the substrate may be formed of silicon, or glass.

In operation 603, a first photo resist (PR) pattern 650 is formed on the substrate. The first PR pattern may define a first hole 652 in which a tile may be disposed.

In operation 605, the first hole, corresponding to a guide portion, is formed by etching the substrate using the first PR pattern as an etching mask. For instance, a height of the first hole may be controlled by etching the substrate to a depth in the range of tens of µm to hundreds of µm.

For instance, a plurality of first holes may be formed in rows and columns to be separated from each other by a predetermined gap. As an example, the plurality of first holes may be formed collinearly, in an x-axial direction or a y-axial direction. Accordingly, a plurality of tiles to be fixed in the plurality of holes may be aligned in uniform directions.

The first hole may be formed to have a width wider than a width of the tile by a predetermined size, for example, 10 µm to 20 µm, whereby the tile may be readily inserted.

Also, when the first hole is formed, a second projection, for example, in a column-shaped projection, may be formed, on the substrate, to separate adjacent first holes. That is, the second projection may refer to a portion remaining between the adjacent first holes, without being etched by the first PR pattern, during a process of forming the first holes on the substrate through an etching process.

In operation 607, a second PR pattern 660 is formed on the first hole. The second PR pattern may define a second hole 662 in which an adhesive material may be disposed.

In operation 609, the second hole, corresponding to an adhesive portion, is formed by etching a portion of the first hole using the second PR pattern as an etching mask. For instance, a height of the second hole may be controlled by etching the first hole to a depth in the range of tens of µm to hundreds of µm.

A plurality of second holes may be formed in a lower portion of the plurality of first holes forming a ledge 664 corresponding to a lower portion of the guide portion. The ledge may be formed to extend continually around the perimeter of the second hole 662 or may be formed to extend from the opposite sides of the first hole 652, forming two ledges. However, the configuration of the ledge is not limited thereto. Similarly to the plurality of first holes, the plurality of second holes may also be formed in rows and columns to be separated from each other by a predetermined gap.

In operation 611, the first PR pattern and the second PR pattern are eliminated. The elimination of the first PR pattern and the second PR pattern may be performed using a method generally known in the art, for example, an ashing process using gas plasma, for example, oxygen gas (O₂), nitrogen gas (N₂), hydrogen gas (H₂), and the like.

Using the semiconductor process technology as described in operations 601 through 611, the first hole, that is, the guide portion, and the second hole, that is, the adhesive portion, may be formed on the substrate, by controlling a size of the first hole and a size of the second hole adroitly in units of µm. The size of the first hole and the size of the second hole may include, for example, a height, and a width. The plurality of first holes and the plurality of second holes may be provided in a form of a matrix including rows and columns, whereby the substrate on which tiles may be aligned in a form of a multi-array may be formed.

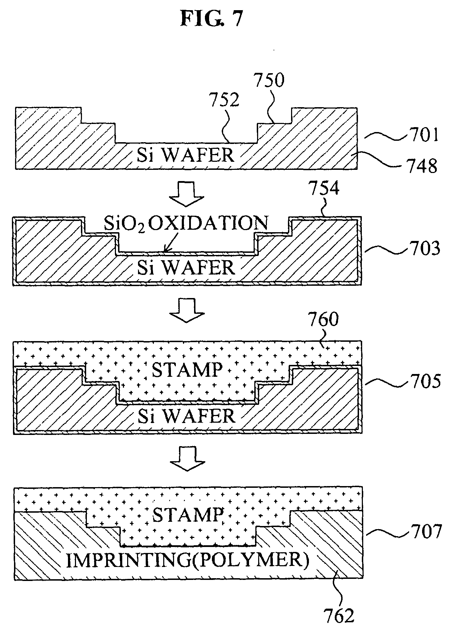

FIG. 7 illustrates another example of a method of manufacturing a substrate of a multi-array ultrasonic probe apparatus.

Referring to FIG. 7, in operation 701, a first substrate 748 including a guide portion 750 and an adhesive portion 752 is provided. The first substrate may refer to a substrate formed by the operations of FIG. 6. As an example, the substrate may be formed of silicon, or glass.

In operation 703, an oxidized layer 754 is formed by oxidation of a surface of the first substrate 748. Operation 703 may be omitted selectively.

In operation 705, a stamp frame 760 is formed by forming an electroplating layer on the first substrate.

In operation 707, a second substrate 762 having an identical shape of the first substrate is formed, by performing imprinting on the second substrate that is formed of a polymer-based material, using the stamp frame as an imprinting jig.

For instance, the substrate may be formed at a relatively low cost or in a relatively short period of time by forming the substrate using imprinting technology, when compared to a substrate formed using the semiconductor process technology.

A number of examples have been described above. Nevertheless, it should be understood that various modifications may be made. For example, suitable results may be achieved if the described techniques are performed in a different order and/or if components in a described system, architecture, device, or circuit are combined in a different manner and/or replaced or supplemented by other components or their equivalents. Accordingly, other implementations are within the scope of the following claims.

The foregoing exemplary embodiments and advantages are merely exemplary and are not to be construed as limiting. The present teaching can be readily applied to other types of apparatuses. Also, the description of the exemplary embodiments is intended to be illustrative, and not to limit the scope of the claims, and many alternatives, modifications, and variations will be apparent to those skilled in the art.

## Claims

1. A multi-array ultrasonic probe apparatus (100), comprising:
n tiles (101, 98) which are configured to transmit and receive ultrasonic beams; and
a substrate (103) comprising n guide portions (105) on which the n tiles are mounted, respectively, to be aligned in a multi-array, wherein the substrate further comprises:
n adhesive portions (107) which are disposed in a lower portion of the n guide portions;
**characterized in that** the substrate further comprises:
n outlets (109) which are configured to discharge, to an outside of the substrate, an excess of an adhesive material disposed in the adhesive portions.

2. The apparatus of claim 1, wherein widths of the n guide portions are wider than widths of corresponding n adhesive portions.

3. The apparatus of claim 1 or 2, wherein each of the n guide portions has identical heights, and
each of the n adhesive portions has identical heights.

4. The apparatus of any one of claims 1 to 3, wherein:
the n guide portions are disposed in a matrix on the substrate with a first predetermined gap between adjacent guide portions to separate the adjacent guide portions from one another, and
the n adhesive portions are disposed in a matrix on the substrate with a second predetermined gap between adjacent adhesive portions to separate the adjacent adhesive portions from one another.

5. The apparatus of any one of claims 1 to 4, wherein widths of the n guide portions are wider than widths of the n tiles by a predetermined size.

6. The apparatus of any one of claims 1 to 5, wherein each of the n tiles comprises:
an Application Specific Integrated Circuit, ASIC, a lower portion of which is disposed proximate a respective adhesive portion, and
a Capacitive Micromachined Ultrasonic Transducer, CMUT, attached to an upper portion of the ASIC.

7. The apparatus of one of claims 1 to 6, wherein the substrate comprises one of silicon, glass, and a polymer-based material.

8. The multi-array ultrasonic probe apparatus (100) of claim 1, wherein the n adhesive portions (107) comprise at least one projection.

9. A method of manufacturing a multi-array ultrasonic probe apparatus (100), the method comprising:
providing a substrate (103) comprising n guide portions (105), wherein the providing comprises:
providing the substrate with n adhesive portions proximate to a lower portion of the n guide portions, and
aligning n tiles (98, 101), which transmit and receive ultrasonic beams, in a multi-array by mounting the n tiles on the n guide portions, respectively;
**characterized by**
providing the substrate with n outlets which discharge, to an outside of the substrate, an excess of an adhesive material disposed in the adhesive portions.

10. The method of claim 9, wherein each of the n tiles comprises an Application Specific Integrated Circuit (ASIC), and a Capacitive Micromachined Ultrasonic Transducer (CMUT) attached to an upper portion of the ASIC, and
the aligning comprises mounting a lower portion of the ASIC onto respective guide portions.

11. A device comprising:
a multi-array ultrasonic probe apparatus according to claim 1, wherein
each guide portion is configured to align each respective tile on the substrate and comprises:
first sides extending from a surface of the substrate substantially perpendicularly with respect to the surface of the substrate, and
a ledge which is configured to extend from the first sides at a first height from the surface of the substrate, substantially perpendicularly with respect to the first sides, and on which each respective tile is mounted.

12. The device of claim 11, wherein each of adhesive portions comprises a cavity comprising:
second sides extending from the ledge, and
a bottom surface disposed at a second height greater than the first height, from the surface of the substrate, which is configured to connect the second sides and attach each respective tile to the substrate;
wherein the outlets are preferably configured to extend from a respective cavity to an outside of the substrate and discharge an excess of the adhesive material which builds up in a respective adhesive portion when the tiles are being mounted onto respective mounting portions.

## Patentansprüche

1. Multi-Array-Ultraschallsondenvorrichtung (100), umfassend:
n Kacheln (101, 98), die konfiguriert sind, um Ultraschallstrahlen zu senden und zu empfangen, und
ein Substrat (103), das n Führungsteile (105) umfasst, an denen die n Kacheln jeweils montiert sind, um in einem Mehrfach-Array ausgerichtet zu werden, wobei das Substrat weiterhin umfasst:
n Klebeteile (107), die in einem unteren Teil der n Führungsteile angeordnet sind,
**dadurch gekennzeichnet, dass** das Substrat weiterhin umfasst:
n Auslässe (109), die konfiguriert sind, um überschüssiges Klebematerial in den Klebeteilen von dem Substrat nach außen auszuführen.

2. Vorrichtung nach Anspruch 1, wobei die Breiten der n Führungsteile breiter sind als die Breiten der entsprechenden n Klebeteile.

3. Vorrichtung nach Anspruch 1 oder 2, wobei die n Führungsteile identische Höhen aufweisen und wobei die n Klebeteile identische Höhen aufweisen.

4. Vorrichtung nach einem der Ansprüche 1 bis 3, wobei:
die n Führungsteile in einer Matrix auf dem Substrat mit einem ersten vorbestimmten Zwischenraum zwischen benachbarten Führungsteilen angeordnet sind, um die benachbarten Führungsteile voneinander zu trennen, und
die n Klebeteile in einer Matrix auf dem Substrat mit einem zweiten vorbestimmten Zwischenraum zwischen benachbarten Klebeteilen angeordnet sind, um die benachbarten Klebeteile voneinander zu trennen.

5. Vorrichtung nach einem der Ansprüche 1 bis 4, wobei die Breiten der n Führungsteile um eine vorbestimmte Größe breiter sind als die Breiten der n Kacheln.

6. Vorrichtung nach einem der Ansprüche 1 bis 5, wobei jede der n Kacheln umfasst:
eine anwendungsspezifische integrierte Schaltung (ASIC), deren unterer Teil in Nachbarschaft zu einem entsprechenden Klebeteil angeordnet ist, und
einen kapazitiven mikromechanischen Ultraschallwandler (CMUT), der an einem oberen Teil der ASIC angebracht ist.

7. Vorrichtung nach einem der Ansprüche 1 bis 6, wobei das Substrat aus Siliziumglas oder einem polymerbasierten Material ausgebildet ist.

8. Multi-Array-Ultraschallsondenvorrichtung (100) nach Anspruch 1, wobei die n Klebeteile (107) wenigstens einen Vorsprung umfassen.

9. Verfahren zum Herstellen einer Multi-Array-Ultraschallsondenvorrichtung (100), wobei das Verfahren umfasst:
Vorsehen eines Substrats (103) mit n Führungsteilen (105), wobei das Vorsehen umfasst:
Versehen des Substrats mit n Klebeteilen in Nachbarschaft zu einem unteren Teil der n Führungsteile, und
Ausrichten von n Kacheln (98, 101), die Ultraschallstrahlen senden und empfangen, in einem Multi-Array durch das Montieren der n Kacheln jeweils an den n Führungsteilen,
**gekennzeichnet durch**
Versehen des Substrats mit n Auslässen, die überschüssiges Klebematerial in den Klebeteilen von dem Substrat nach außen ausführen.

10. Verfahren nach Anspruch 9, wobei jede der n Kacheln eine anwendungsspezifische integrierte Schaltung (ASIC) und einen kapazitiven mikromechanischen Ultraschallwandler (CMUT), der an dem oberen Teil der ASIC angebracht ist, umfasst, und
das Ausrichten das Montieren eines unteren Teils der ASIC an entsprechenden Führungsteilen umfasst.

11. Vorrichtung, die umfasst:
eine Multi-Array-Ultraschallsondenvorrichtung nach Anspruch 1, wobei:
jeder Führungsteil konfiguriert ist, um jede entsprechende Kachel an dem Substrat auszurichten, und umfasst:
erste Seiten, die sich von einer Oberfläche des Substrats im Wesentlichen senkrecht in Bezug auf die Oberfläche des Substrats erstrecken, und
eine Kante, die konfiguriert ist, um sich von den ersten Seiten mit einer ersten Höhe von der Oberfläche des Substrats im Wesentlichen senkrecht in Bezug auf die ersten Seiten zu erstrecken, und an der jede entsprechende Kachel montiert ist.

12. Vorrichtung nach Anspruch 11, wobei jeder Klebeteil einen Hohlraum umfasst, der umfasst:
zweite Seiten, die sich von der Kante erstrecken, und
eine Bodenfläche, die auf einer zweiten Höhe, die größer als die erste Höhe ist, von der Oberfläche des Substrats angeordnet ist und konfiguriert ist, um die zweiten Seiten zu verbinden und jede entsprechende Kachel an dem Substrat anzubringen,
wobei die Auslässe vorzugsweise konfiguriert sind, um sich von einem entsprechenden Hohlraum von dem Substrat nach außen zu erstrecken und das überschüssige Klebematerial auszuführen, das sich in dem entsprechenden Klebeteil aufbaut, wenn die Kacheln an den entsprechenden Montageteilen montiert werden.

## Revendications

1. Appareil à sonde ultrasonore multiréseau (100), comprenant :
n carreaux (101, 98) qui sont configurés pour transmettre et recevoir des faisceaux ultrasonores ; et
un substrat (103) comprenant n portions guides (105) sur lesquelles les n carreaux sont respectivement montés pour être alignés dans un multiréseau, dans lequel le substrat comprend en outre :
n portions adhésives (107) qui sont agencées dans une portion inférieure des n portions guides ;
**caractérisé en ce que** le substrat comprend en outre :
n sorties (109) qui sont configurées pour décharger, vers l'extérieur du substrat, un excès de matériau adhésif agencé dans les portions adhésives.

2. Appareil selon la revendication 1, dans lequel les largeurs des n portions guides sont supérieures aux largeurs de n portions adhésives correspondantes.

3. Appareil selon la revendication 1 ou 2, dans lequel chacune des n portions guides présente des hauteurs identiques, et
chacune des n portions adhésives présente des hauteurs identiques.

4. Appareil selon l'une quelconque des revendications 1 à 3, dans lequel :
les n portions guides sont agencées dans une matrice sur le substrat avec un premier intervalle prédéterminé entre des portions guides adjacentes pour séparer les portions guides adjacentes les unes des autres, et
les n portions adhésives sont agencées dans une matrice sur le substrat avec un deuxième intervalle prédéterminé entre des portions adhésives adjacentes pour séparer les portions adhésives adjacentes les unes des autres.

5. Appareil selon l'une quelconque des revendications 1 à 4, dans lequel les largeurs des n portions guides sont supérieures aux largeurs des n carreaux d'une taille prédéterminée.

6. Appareil selon l'une quelconque des revendications 1 à 5, dans lequel chacun des n carreaux comprend :
un circuit intégré à application spécifique ASIC, soit Application Specific Integrated Circuit, dont une portion inférieure est agencée à proximité d'une portion adhésive respective, et
un transducteur ultrasonore micro-usiné capacitif CMUT, soit Capacitive Micromachined Ultrasonic Transducer, attaché à une portion supérieure de l'ASIC.

7. Appareil selon l'une des revendications 1 et 6, dans lequel le substrat comprend un matériau parmi le silicium, le verre et un matériau à base polymère.

8. Appareil à sonde ultrasonore multiréseau (100) selon la revendication 1, dans lequel les n portions adhésives (107) comprennent au moins une projection.

9. Procédé de fabrication d'un appareil à sonde ultrasonore multiréseau (100), le procédé comprenant :
la fourniture d'un substrat (103) comprenant n portions guides (105), dans lequel la fourniture comprend :
la fourniture au substrat de n portions adhésives à proximité d'une portion inférieure des n portions guides, et
l'alignement de n carreaux (98, 101), qui transmettent et reçoivent des faisceaux ultrasonores dans un multiréseau en montant respectivement les n carreaux sur les n portions guides ;
**caractérisé par**
la fourniture au substrat de n sorties qui déchargent, vers l'extérieur du substrat, un excès de matériau adhésif agencé dans les portions adhésives.

10. Procédé selon la revendication 9, dans lequel chacun des n carreaux comprend un circuit intégré à application spécifique (ASIC) et un transducteur ultrasonore micro-usiné capacitif (CMUT) attaché à une portion supérieure de l'ASIC, et
l'alignement comprend le montage d'une portion inférieure de l'ASIC sur des portions guides respectives.

11. Dispositif comprenant :
un appareil à sonde ultrasonore multiréseau selon la revendication 1, dans lequel
chaque section de guidage est configurée pour aligner chaque carreau respectif sur le substrat et comprend :
des premiers côtés qui s'étendent depuis une surface du substrat sensiblement perpendiculairement à la surface du substrat, et
un rebord qui est configuré pour s'étendre depuis les premiers côtés à une première hauteur depuis la surface du substrat, sensiblement perpendiculairement aux premiers côtés, et sur lequel chaque carreau respectif est monté.

12. Dispositif selon la revendication 11, dans lequel chacune des portions adhésives comprend une cavité qui comporte :
des deuxièmes côtés qui s'étendent depuis le rebord, et
une surface de fond agencée à une deuxième hauteur supérieure à la première hauteur, depuis la surface du substrat, qui est configurée pour connecter les deuxièmes côtés et attacher chaque carreau respectif au substrat ;
dans lequel les sorties sont de préférence configurées pour s'étendre depuis une cavité respective vers l'extérieur du substrat et pour décharger un excès de matériau adhésif qui s'accumule dans une portion adhésive respective quand les carreaux sont montés sur des portions de montage respectives.
